Europäisches Patentamt

European Patent Office

Office européen des brevets

(11) Publication number: **0 315 627 B1**

# EUROPEAN PATENT SPECIFICATION

(45) Date of publication of patent specification: **19.08.92**   (51) Int. Cl.5: **A61K  31/19**, A61K 33/14, A61L 2/16

(21) Application number: **87901862.0**

(22) Date of filing: **06.02.87**

(86) International application number:
**PCT/US87/00288**

(87) International publication number:
**WO 88/01507 (10.03.88 88/06)**

(54) **A METHOD TO TREAT BLOOD.**

(30) Priority: **01.08.86 US 892058**

(43) Date of publication of application:
**17.05.89 Bulletin  89/20**

(45) Publication of the grant of the patent:
**19.08.92 Bulletin  92/34**

(84) Designated Contracting States:
**AT BE CH DE FR GB IT LI LU NL SE**

(56) References cited:
**BE-A- 895 860**

**SARIN et al, The New England Journal of
Medicine, p.1416, Vol.221, Nov.28, 1985**

(73) Proprietor: **RUBINSTEIN, Alan I.**
**100 S. Doheney Drive Number 301**
**Los Angeles, CA 90048(US)**

(72) Inventor: **RUBINSTEIN, Alan I.**
**100 S. Doheney Drive Number 301**
**Los Angeles, CA 90048(US)**

(74) Representative: **Hedley, Nicholas James Matthew et al**
**Stephenson Harwood One, St. Paul's
Churchyard**
**London EC4M 8SH(GB)**

EP 0 315 627 B1

## Description

Units of blood, both red blood cells (RBC units) and units of plasma, have a well known risk for tramsitting Hepatitis B virus, non-A, non-B Hepatitis virus(es), and HTLV-III (HIV-1) virus. HTLV-III (HIV-1) is a human retrovirus which has been implicated in the causation of AIDS (See Gallo, R.C. et al.) Frequent detection and isolation of cytopathic retroviruses [HTLV-III] from patients with AIDS and at risk for AIDS. Science 1984; 224:500-3. Also see: Savin, P.S. et al., Human T-Lymphotropic Retroviruses I Adult T-cell Leukemia - Lymphoma And Acquired Immune Deficiency Syndrome, J. Clinical Immunol., 1984; 4:415-23. Wong-Staal F., Gallo, R.C., Human T-Lymphotropic Retroviruses, Nature 1985; 317:395-402). There is titus an important need to make units of blood safe for transfusion, that is to less the probability of transferring HTLV-III by blood transfusion would be of great importance.

The present invention provides a method of treating substances comprising red blood cells and this method is defined in the following claims.

## Example

A solution (disinfectant) was prepared as follows: LD$^{tm}$ disinfectant was obtained from Alcide Corporation and was mixed as follows: One part LD base was mixed with ten parts normal saline, one part activator was added and diluted with normal saline to dilution of 1:200. This solution is composed of sodium chlorite and lactic acid. The pH of the final solution (disinfectant) was adjusted to between 4 - 5. The final solution contained sodium chlorite and lactic acid, both at concentration below 2%.

Two aliquats of red blood cells from an American Red Cross unit of red blood cells was washed three times in normal saline. One aliquat of the washed red blood cells (RBC) was labeled Experiment (EXP) and took up approximately 1/3 of a tube; the tube was filled with the above prepared distinfectant, mixed and allowed to stand for approximately 10-50 seconds. Then the cells were washed in normal saline four times, and resuspended in normal saline, no hemolysis was observed as compared to the control tube of RBC's which was washed identically as the experiment but to which no disinfectant was added.

In order to verify that the RBC's treated with the disinfectant were suitable for transfusion an assay was performed on them following several days storage in normal saline at approximately 4° centigrade. Thus RBC 2, 3, DPG (hereinafter referred to as 2,3 DPG) and RBC ATP was performed following 37° C incubation in a solution of glucose, inorganic phhophorus, potassium and magnesium.

The result of these assays, the methodology used, was as described (see Keith, A.S.: Reduced nicotinamide ademine denucleotide-linked analysis of 2,3 diphophoglyceric acid: spectrophotometric and fluorometric procedures, General Lab, Clin. Med., 77:470, 1971; see also see: J.A. Worek, D. Gruber, W., Bergneyer, H.U.: Adenosine, 5', triphosphate, determination with 3-phosphoglycerate kinase, in Bergmeyer, H.U. (ed): Methods of Enzymatic Analysis, Vol. 4, N.Y. Acadeic Press, 1974, p. 2097).

The presence of 2,3-diphosphoglyceric acid (DPG) phosphatase activity in preparations of monophosphoglycerate mutase (PGM) from muscle and other sources has been noted by several workers and was exploited by Lowry and co-workers to measure the very low levels of 2,3-DPG in acid extracts of brain tissue. The products of the phosphatase, 3-phosphalglyceric acid (3-PGA), was measured fluorometrically in a reduced nicotinamide adenine dinucleotide (NADH)-linked reaction in which 3-PGA was converted stoichiometrically to glyceraldehyde 3-phosphate (G-3P) by phosphoglycerate kinase (PGK) and glyceraldehyde 3-phosphate dehydrogenase (G3PD). This fluorometric procedure was modified by the author for measurement of the much higher concentrations of 2,3-DPG in red cells.

Rose has reported that red cells 2,3-DPG phosphatase is stimulated by both pyrophosphate and 2-phosphoglycolic acid, a property which is shared by the phosphatase activity in muscle PGM. She has devised a spectrophotometric assay for 2,3-DPG with the use of 2-phosphoglycoate and PGM, converting the 3-PGA formed to lactate. The fluorometric and spectrophotometric assays for 2,3-DPG described here are those which proved most versatile and reliable; they represent a composite of the methods of Lowry and co-workers, Rose and Liebowitz, and Czok and Eckert.

## Materials and Methods

Imidazole (grade III), reduced glutathione (GSH), and hydrazine sulfate solution (No. 750-3) were obtained. 2-Phosphoglycolate was also obtained. Distilled water was passed through a mixed bed deionizer which greatly reduced its fluorescence.

Preparation of samples: Customarily 1. Vol. of whole blood is added to 2 Vol. of ice-cold 6% (w/v) perchoric acid (PCA), mixed thoroughly, and left on ice for at least 15 minutes. The brown denatured protein is separated by centrifugation at 27,000 x g for 20 minutes at 2° C. The clear supernatant is neutralized with approximately 1/6 Vol. of 2M $KHCO_2$.

Spectrophotometric assay: All reagents are prepared as stock solutions and stored frozen, except imidazole and hydrazine which are stored at room temperature. Reactions are performed at 25 to 28° C (without temperature control) in an automatic recording spectrophometer in quartz semi-microcuvettes with 1.0 cm path lengths at 340 $\mu$m. For normal whole blood, neutral PCA extract (0.1 ml) is added to 1.0 ml aliquots of the reaction mixture. The volume of extract can be increased to at least 0.3 ml without affecting the reaction mixture. The combined solution of G3PD and PGK (in r $\mu$l) is added after all the 3-PGA and 1,3-DPG has reacted (usually they are undetectable), the absorbance at 340 $\mu$m. is determined and PGM (in 5 $\mu$l) is added. The reaction normally goes to completed in 15 to 20 minutes, although this should be determined for each new set of reagents as the phosphatase activity of different lots of PGM varies slightly. The blank cuvette contains distilled water instead of PCA extract. Blanks containing PCA extract, but no enzymes, are usually identical, but this should be verified periodically. When 2-mercaptoethanol was substituted for GSH, sporadically high blanks (.03 to .04 O.D. units per 10 minutes) were noted in the presence of neutral PCA extracts of whole blood without any added enzymes.

Fluorometric assay: A sample size of PCA extract (2 to 5 $\mu$l) is employed depending on the hematocrit of the sample. Samples and standards are added with the same selected micropipette. The reaction is complete in 5 minutes.

Calculations: 2,3-DPG concentration is derived by determining the difference in absorbance at 340 $\mu$m before and after adding PGM corrected for the reagent blank (usually less than 0.010 O.D. units) with an extinction coefficient of 6.22 O.D. units per millimole of NADH in the fluorometric assay, the change in NADH fluorescence is compared with that of a 2,3 Def solution which has been standardized spectrophotometrically.

There is significant deviation from linearity in the absence of hydrazine when the initial concentration of 2,3-DPG approaches that of the available NADH. The obligatory liberation of inorganic phosphate from 2-3-DPG adversely affects the final ratio of 1,3-DPG to G-3-P so that G-3-P must be trapped with hydrazine, unless a large excess of NADH is employed. The potency of the hydrazine solution, unless freshly prepared, should be verified periodically by checking the linearity of a standard curve.

The half time of the fluorometric assay is normally less than 1 minute. Because of its great sensitivity, very low concentrations of 2,3-DPG as might occur during blood storage or in vitro experiments, can be easily measured. The accuracy of this method is determined largely by the quality of the micropipettes and the skill of the technician in handling them.

Specificity: The addition of PGM to the system results in the conversion of both 2,3-DPG and 2-PGA to 3-PGA. In normal blood the concentration of 2-PGA is 300 times less than that of 2,3-DPG and can be ignored. However, under conditions where these compounds are more nearly equal (as in most tissues) discrimination may be more important. The assay can be modified such that an approximation of 2-PGA content can be obtained by adding PGM (5 $\mu$g per milliliter) without phosphoglycolate after 3-PGA has reacted. Under these conditions, 2-PGA will be rapidly converted to 3-PGA, while 2,3-DPG will react very slowly. Selective activation of the phosphatase with phosphoglycolate will then measure 2,3-DPG. This modification requires fluorometric measurements of NDAH for sufficient sensitivity at the low levels of 2-PGA usually present in tissues.

The advantages of this method are several. Its most important aspect is that it is an "endpoint" assay rather than a rate assay. The assay methods which relate 2,3-DPG concentration to its catalytic effect on the PGM reaction involve rate measurements which are much more likely to be affected by minor variations in assay conditions. "Rate" in these methods is often determined by an initial reading followed by a single additional reading at one time intervals assuming linearity; any deviations from linearity among different samples will therefore be undetected. Furthermore, the calculations depend on a standard curve of known purity.

The "endpoint" assay on the contrary may be read at any convenient time after going to completion provided that the blank cuvette is read at a similar interval. The calculations are based on the molar extinction coefficient of NADH when determined spectrophotometrically and thus do not depend on a standard curve. If greater sensitivity is required, the fluorometric assay can be used. No fractionation of the extracted material is required as in the total phosphate and chromotropic acid methods. The specificity, although not absolute because of the co-reaction of 2-PGA, is entirely adequate for most red cell applications where the 2-PGA concentration is negligible. Selective activation of 2,3-PGA activity by phosphoglycolate after 20-PGA has reacted can improve the specificity of the assay.

The "backward" reaction to G3P is more versatile than the "forward" reaction to lactate because the concentrations of 3-PGA and 2-PGA are usually negligible compared to that of 2,3-DPG, whereas pyruvate may accumulate significantly under certain experimental conditions and the initial lactate dehydrogenase reaction may then exhaust much of the NADH. Furthermore, at very low concentrations of 2,3-DPG, the co-reaction of 2-PGA concentration exceeds that of 2-PGA by a factor of 5 to 10 in the intact cell.

## RESULTS

The RBC ATP and 2,3-DPG of the sample of RBC treated with the disinfectant (Experiment) when compared to the sample of RBC's not treated showed no significant difference in their ability to regenerate RBC ATP and 2,3-DPG following incubation. These two enzymes (see References) are considered important predictors of RBC viability and suitability for transfusion. It is thus concluded that application of the disinfectant solution as prepared according to the method as described here and following washing with normal saline, the RBC's are suitable for transfusion, and these RBC's carry a much lessened risk (or total absence of risk) of transmitting AIDS or other viral diseases.

The results following incubation of human plasma are listed in Table I.

## TABLE I.

The incubation experiments to determine the ability of the red cells to regenerate ATP and 2,3-DPG gave the following results:

|  | Control | | Experiment | |
|---|---|---|---|---|
|  | ATP | 2,3-DPG | ATP | 2,3-DPG |
| 0 h | 0 | 0 | 0 | 0 |
| 2 h | 1.79 | 0 | 1.34 | 2.89 |
| 4 h | 1.19 | 0 | 1.34 | 0 |

(Results are expressed in moles/gram Hb)

The incubations were performed at 37° C with the supplementation of glucose, inorganic phosphorus, potassium, and magnesium.

## TABLE II

[Treatment of washed quantity of RBC's for approximately 4 minutes with the same solution as used for Table I, then the cells were washed in normal saline.]

The incubation experiments to determine the ability of the red cells to regenerate ATP        ‎ gave the following results:

|  | Control | Experiment |  |
|---|---|---|---|
|  | ATP | ATP |  |
| 0 h | 3.83 | 5.28 | (Results are ex- |
| 2 h | 4.35 | 4.26 | pressed in umoles/ gram Hb) |

The incubations were performed at 37° C with the supplementation of glucose, inorganic phosphorus, potassium, and magnesium.

It should be noted that following 4 minutes of contact with the disinfectant there was no evidence for hemolysis and no loss of ATP.

In the past, it has not been possible to treat units of red blood cells with disinfectant, as this treatment has been too toxic for the cells. However the development of the new disinfectants as well as automated cell washers (e.g. IBM 2292 cell washer) to wash units of red blood cells has opened up a new avenue in regard to sterilizing units for transfusion.

It has been reported that a laboratory disinfectant composed of approximately 0.23% sodium chlorite and 1.26% lactic acid (LD[tm] Alcide, Norwalk, Conn.) can completely inactivate HTLV-III/LAV virus (see Sarin, P.S. et al., NEJM Vol. 33, No. 221, p.1416, 28.11.1985) at a dilution of 1:200 or less.

It is thus the intention of the inventor to describe a method, for example, whereby units of human red blood cells may first be separated from plasma and thereafter be exposed to a solution composed of approximately 0.23% Sodium Chlorite and 1.26% lactic acid (and then this solution may be diluted with 0.9% NaCl up to 1:200) for a very short time (for example, a few seconds or minutes[s]) and thereafter the unit of red blood cells may be washed, for example using an automated cell washer (e.g. IBM). And then following the washing in normal saline, the red blood cells are now ready and safe for transfusion, safe from transmitting AIDS, as well as other viruses which may be in blood, for example Hepatitis B and non-A, non-B hepatitis virus(es) and LMV virus and Epstein-Barr virus.

It has now been demonstrated that units of blood (red blood cells) may be safely, economically sterilized while in a blood collection bag. The entire process may for example be automated using for example (IBM Cell Washer) an automated cell washer. Thus the cell washer may be programmed to give the disinfectant at least a 200-fold dilution of its standard use dilution, let the disinfectant mix with the unit of red cells for a few seconds and then automatically wash the cells. This automation increases the utility of the invention, since now units may be washed rapidly and can now be applied on a massive scale.

## Claims

1. A method of reducing the infectivity of microorganisms in substances comprising red blood cells, said method comprising the steps of:

   (1) preparing a disinfectant by forming a solution of lactic acid and sodium chlorite in normal saline such that the pH is approximately that of saline, the concentration of the disinfectant being sufficient to reduce the infectivity of the said microorganisms,

   (2) exposing the substance comprising red blood cells to the said solution for a time sufficient to inactivate the said microorganism and

   (3) washing the red blood cells with normal saline until the concentration of disinfectant is insignificant.

2. A method as claimed in claim 1, whereby the said substance is composed of red blood cells.

3. A method as claimed in claim 1 or claim 2, wherein the lactic acid and sodium chlorite are each present in the solution in a concentration of less than two percent.

4. A method as claimed in claim 1, wherein the microorganism is a virus, e.g. HIV-1 virus or a hepatitis virus, such as Hepatitis B virus, Hepatitis non-A, non-B, Hepatitis delta agent (virus) or Hepatitis A.

5. A method as claimed in claim 1, wherein the disinfectant is composed of approximately 0.23 percent sodium chlorite and 1.26 percent lactic acid diluted with normal saline or a solution of approximately 0.9% NaCl.

6. A method as claimed in claim 5, wherein the solution of disinfectant contains from $1.15 \times 10^{-4}$ percent sodium chlorite and $6.3 \times 10^{-4}$ percent lactic acid to 0.23 percent sodium chlorite and 1.26 percent lactic acid.

7. A method as claimed in claim 1, whereby the disinfectant is kept in contact with the substance for a time period of from 0.5 seconds to 30 minutes, e.g. from 0.2 minutes to 5 minutes, and preferably the substance is shaken while in contact with the disinfectant.

8. A method claimed in any one of claims 1 to 7, wherein the exposing step is performed in an automated or semi-automated cell washer.

9. A method to reduce infectivity of HIV-1 virus that causes AIDS, said method comprising:

   (a) preparing a disinfectant composed of lactic acid and sodium chlorite, both in concentrations of less than 2% and both in a solution of normal saline, and

   (b) exposing a substance comprising red blood cells to the disinfectant for a time period sufficient to inactivate the HIV-1 virus and then

   (c) washing the red blood cells with normal saline sufficiently such that the disinfectant is not significantly present.

## Patentansprüche

6

1. Verfahren zur Verringerung der Infektionswirkung von Mikroorganismen in Substanzen, die rote Blutkörperchen enthalten, wobei das Verfahren folgende Schritte umfaßt:

(1) Herstellung eines Entkeimungsmittels durch Bildung einer Lösung aus Milchsäure und Natriumchlorid in Neutralsalz, so daß der pH-Wert annähernd den des Salzes hat, und daß die Konzentration des Entkeimungsmittels genügt, um die Infektionswirkung besagter Mikroorganismen zu verringern,

(2) Zusammenbringen der rote Blutkörperchen enthaltenden Substanz mit der genannten Lösung für eine Zeit, die ausreicht, die besagten Mikroorganismen zu inaktivieren, und

(3) Waschen der roten Blutkörperchen mit Neutralsalz, bis die Konzentration des Entkeimungsmittels unbedeutend ist.

2. Verfahren nach Anspruch 1,
wobei die genannte Substanz aus roten Blutkörperchen zusammengesetzt ist.

3. Verfahren nach Anspruch 1 oder Anspruch 2,
wobei die Milchsäure und Natriumchlorid jeweils in einer Konzentration von weniger als 2 % in der Lösung enthalten sind.

4. Verfahren nach Anspruch 1,
wobei der Mikroorganismus ein Virus ist, zum Beispiel HIV-I-Virus oder ein Hepatitis-Virus, wie Hepatitis-B-Virus, Hepatitis-non-A, Hepatitis-non-B, Hepatitis-delta-Agent (Virus) oder Hepatitis-A.

5. Verfahren nach Anspruch 1,
wobei das Entkeimungsmittel zusammengesetzt ist aus annähernd 0,23 % Natriumchlorid und 1,26 % Milchsäure, gelöst in Neutralsalz oder einer Lösung aus annähernd 0,9 % NaCl.

6. Verfahren nach Anspruch 5,
wobei die Lösung des Entkeimungsmittels von $1,15 \times 10^{-4}$ % Natriumchlorid und $6,3 \times 10^{-4}$ % Milchsäure bis 0,23 % Natriumchlorid und 1,26 % Milchsäure enthält.

7. Verfahren nach Anspruch 1,
wobei das Entkeimungsmittel mit der Substanz in Kontakt gehalten wird für eine Zeitdauer von 0,5 Sekunden bis 30 Minuten, zum Beispiel von 0,2 Minuten bis 5 Minuten, und wobei die Substanz vorzugsweise geschüttelt wird, während sie in Kontakt mit dem Entkeimungsmittel ist.

8. Verfahren nach einem der Ansprüche 1 bis 7,
wobei der Verfahrensschritt des Zusammenbringens in einer automatischen oder halbautomatischen Zellwaschmaschine durchgeführt wird.

9. Verfahren zur Verringerung der Infektionswirkung von HIV-I-Virus, welches AIDS verursacht, wobei das Verfahren folgende Schritte aufweist:

(a) die Herstellung eines aus Milchsäure und Natriumchlorid zusammengesetzten Entkeimungsmittels, wobei beide Bestandteile in Konzentrationen von weniger als 2 % und beide in einer Neutralsalzlösung vorliegen,

(b) das Zusammenbringen einer rote Blutkörperchen enthaltenden Substanz mit dem Entkeimungsmittel für eine Zeitdauer, die ausreicht, um den HIV-I-Virus zu inaktivieren, und dann

(c) das Waschen der roten Blutkörperchen mit Neutralsalz so ausreichend, daß das Entkeimungsmittel nur unbedeutsam vorhanden ist.

**Revendications**

1. Procédé de réduction du pouvoir d'infection de microorganismes dans des substances comprenant des hématies, ledit procédé comprenant comme étapes :

(1) la préparation d'un désinfectant en formant une solution d'acide lactique et de chlorite de sodium dans du sérum physiologique normal telle que le pH soit approximativement celui de sérum physioloqique, la concentration du désinfectant étant suffisante pour réduire le pouvoir d'infection desdites microorganismes,

(2) l'exposition de la substance comprenant des hématies à ladite solution pendant une durée suffisant à inactiver lesdits microorganismes et

(3) le lavage des hématies avec du sérum physiologique normal jusqu'à ce que la concentration en désinfectant soit insignifiante.

2. Procédé selon la revendication 1, dans lequel ladite substance est composée d'hématies.

3. Procédé selon la revendication 1 ou 2, dans lequel l'acide lactique et le chlorite de sodium sont chacun présents dans la solution à une concentration inférieure à deux pour-cent.

4. Procédé selon la revendication 1, dans lequel le microorganisme est un virus, par exemple le virus HIV-1 ou un virus d'hépatite, tel que le virus d'hépatite B, d'hépatite non-A non-B, l'agent delta d'hépatite (virus) ou d'hépatite A.

5. Procédé selon la revendication 1, dans lequel le désinfectant est constitué d'approximativement 0,23 pour-cent de chlorite de sodium et de 1,26 pour-cent d'acide lactique dilué à l'aide de sérum physiologique normal ou d'une solution d'approximativement 0,9 % NaCl.

6. Procédé selon la revendication 5, dans lequel la solution de désinfectant renferme de $1,15 \times 10^{-4}$ pour-cent de chlorite de sodium et $6,3 \times 10^{-4}$ pour-cent d'acide lactique à 0,23 pour-cent de chlorite de sodium et 1,26 pour-cent d'acide lactique.

7. Procédé selon la revendication 1, dans lequel le désinfectant est maintenu en contact avec la substance pendant une durée comprise entre 0,5 seconde et 30 minutes, par exemple entre 0,2 minute et 5 minutes, et de preférence la substance est agitée tandis qu'elle est en contact avec le désinfectant.

8. Procédé selon l'une quelconque des revendications 1 à 7, dans lequel l'étape d'exposition est accomplie dans un laveur de cellules automatique ou semi-automatique.

9. Procédé de réduction du pouvoir d'infection du virus HIV-1 causant le SIDA, ledit procédé consistant :
(a) à préparer un désinfectant composé d'acide lactique et de chlorite de sodium, tous deux en concentration inférieure à 2 pour-cent et tous deux en solution dans du sérum physiologique normal et,
(b) à exposer une substance comprenant des hématies au désinfectant pendant une durée suffisant à inactiver le virus HIV-1, puis
(c) à laver suffisamment les hématies avec du sérum physiologique normal pour que le désinfectant n'ait plus de présence significative.